Europäisches Patentamt

European Patent Office

Office européen des brevets

(11) Publication number: 0 298 450

A2

# (12) EUROPEAN PATENT APPLICATION

(21) Application number: 88110787.4

(22) Date of filing: 06.07.88

(51) Int. Cl.⁴: **C12P 21/00 , A61K 39/395 , C12N 15/00 , G01N 33/577**

The applicant has filed a statement in accordance with Rule 28 (4) EPC (issue of a sample only to an expert). Accession number of the deposit: ECACC 87062301.

The microorganism has been deposited with the European Collection of Animal Cell Cultures ECACC, Porton, Devon under number ECACC 87062301.     .

(30) Priority: 07.07.87 EP 87109791

(43) Date of publication of application:
**11.01.89 Bulletin 89/02**

(84) Designated Contracting States:
**AT BE CH DE ES FR GB GR IT LI LU NL SE**

(71) Applicant: **Max-Planck-Gesellschaft zur Förderung der Wissenschaften e.V.
Bunsenstrasse 10
D-3400 Göttingen(DE)**

(72) Inventor: **Mölling, Karin, Prof. Dr.
Ihnestrasse 43
D-1000 Berlin 33(DE)**

(74) Representative: **Vossius & Partner
Siebertstrasse 4 P.O. Box 86 07 67
D-8000 München 86(DE)**

(54) Hybridoma cell lines, monoclonal antibodies and their use for immunocytochemical detection or fluorescence-activated sorting of cells expressing the c-myb protein and pharmaceutical compositions.

(57) Described is a hybridoma cell line expressing a monoclonal antibody recognizing a particular hydrophilic epitope conserved in the viral and human cellular myb protein. It allows the immunocytochemical detection of c-myb protein expression in human tissues or cells or cell lines and the fluorescence activated cell-sorting of c-myb expressing cells and the detection in indirect immunoprecipitation, ELISA and Western-Blots.

EP 0 298 450 A2

# Hybridoma Cell Lines, Monoclonal Antibodies and Their Use for Immunocytochemical Detection or Fluorescence-Activated Sorting of Cells Expressing the c-myb Protein and Pharmaceutical Compositions

Avian myeloblastosis virus (AMV, like other acutely transforming viruses, arose by recombination between its helper virus and host cellular DNA-sequences. The transduced DNA-sequence, v-myb, which is derived from the avian c-myb proto-oncogene, is responsible for the oncogenic properties of the virus. AMV causes acute myeloblastic leukaemia in chickens and transforms haematopoietic cells in vitro, but does not induce morphological transformation of cultured fibroblasts. This suggests that only a restricted target-cell population is responsive to its transforming gene product. The v-myb oncogene codes for the protein of 48,000 molecular weight, i.e. $p48^{v-myb}$. AMV is not the only v-myb oncogene-containing virus. The avian leukaemia virus E26 is another replication-defective oncogenic retrovirus whose genome contains a smaller internal segment of the avian c-myb gene than AMV. Oncogenesis effected by E26 appears to be mediated by a single protein, $p135^{gag-myb-ets}$. The myeloid leukaemogenesis shared by E26 and AMV correlates with the common v-myb sequence while the distinct erythroid leukaemogenicity of E26 correlates with v-ets.

Proto-oncogenes generally seem to be involved in intracellular events that regulate cell proliferation and/or differentiation. In particular the nuclear proto-oncogenes c-myc, c-myb, and c-fos may have a regulatory role in gene expression and replication presumably by interaction with cellular DNA.

Activation of the cellular proto-oncogene c-myb is thought to involve either the structural alteration of the coding region that results in the synthesis of an altered gene product or the enhanced expression of a proto-oncogene caused by alterations in its regulatory elements. DNA rearrangements and altered expression of the c-myb proto-oncogene have been reported in mouse plasmocytoid lymphosarkomas, human myeloid and colon tumors.

The c-myb proto-oncogene, the cellular homologue of the transforming gene v-myb of the avian myeloblastosis virus, AMV, is expressed in haematopoietic tissues, particularly those that are enriched for early stages of haematopoietic cell differentiation. Analysing human tumor cell lines, elevated levels of c-myb transcripts have been found almost exclusively in haematopoietic precursor cell lines derived from the T-cell and granulocytemacrophage lineages, with the exception of an aberrant expression of an amplified human c-myb gene in a cell line from a human colon adenocarcinoma (Colo 205). The human c-myb expressing cell line HL-60, established from a patient with acute promyelocytic leukaemia can be induced in vitro to differentiate into mature granulocytes, which is paralleled by a decrease of c-myb mRNA expression. Analysing proto-oncogene transcripts in various types of fresh human tumors the c-myb gene was found to be significantly expressed only in haematopoietic malignancies with the highest c-myb mRNA levels occurring in myeloid and lymphoid leukaemias. In a more recent study with AML patients a correlation was suggested between the level of c-myb transcripts and the peripheral blast count. These results indicate that the c-myb gene may be developmentally regulated and expressed at high levels at early stages of differentiation in the haematopoietic system.

In addition, c-myb is expressed in association with cell proliferation in various cell types. Unlike c-myc and c-fos, c-myb is not involved in immediate response of quiescent chicken embryo fibroblasts to serum stimulation, but does undergo an increase in expression that appears to correlate with cell cycle progression. Similarly, phytohaemagglutinin stimulation of resting human T-lymphocytes results in a marked increase of c-myb mRNA expression which is preceded by a much earlier expression of c-myc mRNA. When synchronized human T-cells were separated at various stages of the cell-cycle, c-myb mRNA was expressed solely during $G_1$ progression, whereas c-myc mRNA increased during the competence phase. In contrast to the thymus where c-myb expression is independent of the cell-cycle and where the high level of c-myb mRNA results from a high absolute rate of transcription presumably from a thymus-specific promoter/enhancer, the variations in c-myb mRNA levels during cellular activation and proliferation seems to be the result of post-transcriptional control.

While most analyses on c-myb expression were restricted to mRNA determination, recent studies have also included protein expression. The c-myb gene product is a molecule of 75 kDa which binds to DNA in vitro like its viral counterpart v-myb. The human c-myb and the v-myb proteins share at least two epitopes, against which cross-reacting monoclonal antibodies have been described. Recent studies on the c/v-myb protein gave the result that it is predominantly located in the nucleus.

Sequence comparison shows that the cellular myb protein contains additional amino acids at the amino-terminal and/or at the carboxy-terminal end when compared with the AMV and E26 v-myb proteins. These deletions probably contribute to the oncogenic potential of the proteins. Analysis of the nucleotide sequence of v-myb and c-myb from various species such as chicken, mouse and man indicates a close homology in

EP 0 298 450 A2

some regions of the genes. These conserved regions are evolutionary stable because they code for important functions of the gene product.

The c-myb proto-oncogene from avian cells codes for a protein of 75,000 molecular weight. Recently the human cellular myb (human c-myb) protein has been identified as a molecule of 80,000 or 75,000 molecular weight.

The present invention relates to hybridoma cell lines secreting monoclonal antibodies recognizing epitopes of the c-myb protein, especially epitopes that are hydrophilic and/or conserved in the c-myb proteins of vertebrates. It also relates to the monoclonal antibodies obtained from these cell lines. We have obtained a hybridoma cell line which is reactive with a conserved hydrophilic epitope present in the avian v-myb and human c-myb proteins. The present invention also relates to the monoclonal antibody obtained from said cell line. Furthermore the invention relates to the immunocytochemical detection of c-myb protein expression in human tissues, cells and cell lines, and to the fluorescence activated cell-sorting of c-myb expressing cells using said monoclonal antibody. The invention also relates to pharmaceutical compositions comprising the monoclonal antibody and a pharmaceutically acceptable carrier.

Isolation of monoclonal antibody against the human c-myb protein.

The EcoRI/XbaI fragment of the v-myb gene from AMV which is derived from the 3'-terminal part of the gene and corresponds to about 70% of the total gene, was cloned into the known thermoinducible expression vector pPLc24 containing a polylinker with cut sites for the restriction enzymes EcoRI, BamHI, HindIII, PstI, BglII, and XbaI. The resulting protein expressed in E.coli is a fusion protein, MS2-v-myb, consisting of the first 99 amino acids of the replicase gene of the bacteriophage MS2 and 269 amino acids of v-myb. It has a molecular weight of 47,000.

The E.coli expressed MS2-v-myb fusion protein was used as antigen for the production of monoclonal antibodies. Any other v-myb or c-myb protein produced by recombinant technology or isolated from tumor cells can serve as antigen for the production of monoclonal antibodies in an analogous way. Strategies and procedures for the preparation of monoclonal antibodies are known; see e.g. Galfré and Milstein, 1981, and Greiser-Wilke et al., 1981. The hybridoma supernatants were screened by ELISA and Western-blot using the MS2-v-myb protein as well as indirect immunoprecipitation using the [$^{35}$S]-methionine metabolically labeled BM-2 and Molt-4 cellular lysates. BM-2 is an AMV-. transformed avian bone marrow cell line and Molt-4 is a human T-lymphoblastic cell line. Positive hybridoma cultures were repeatedly subcloned by end point dilution (normally three times) until they were stable in culture. Only one out of more than a dozen monoclonal antibodies, clone 4/14, recognized the viral as well as the human c-myb protein. The immunoglobulin subgroup of this clone was identified as IgG$_{2B}$ by means of commercially available subgroup-specific antisera.

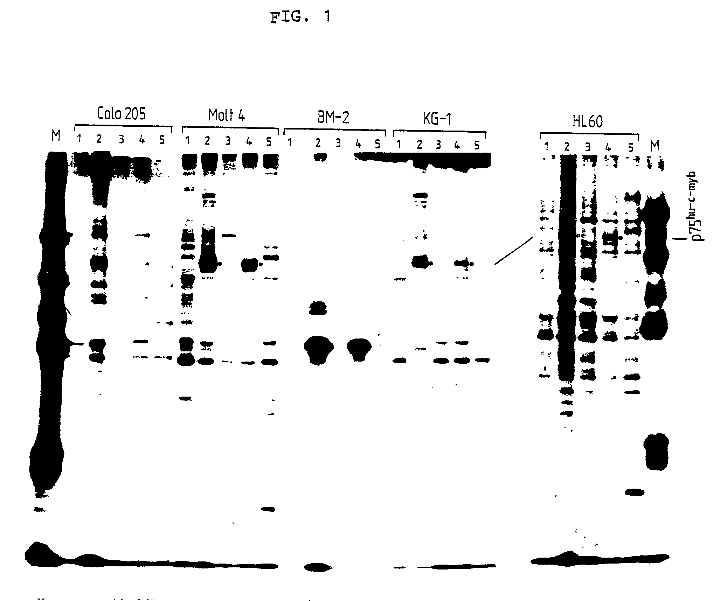

When lysates from [$^{35}$S]-methionine metabolically labeled human lymphoid or myeloid cell lines Molt-4, HL-60, and KG-1 or from the human colon adenocarcinoma cell line Colo 205 were treated with cl 4/14 hybridoma supernatant or supernatants of any other c-myb specific hybridoma in an indirect immunoprecipitation analysis, the p75c-myb protein is detectable (Fig.1). In competition experiments using an excess of bacterially expressed myb protein to block the monoclonal antibody, the immunoprecipitation of p75c-myb is prevented, demonstrating the specificity of the reaction (Fig. 1). For comparison with expression of v-myb, AMV-transformed bone marrow cells, established as permanent line BM-2, were metabolically labeled and treated in parallel. Using standardized amounts of cellular protein for the precipitation study, the amount of the p48v-myb protein is expressed to higher levels in BM-2 cells than the p75c-myb proteins in the human tumor cell lines. Polyvalent rabbit sera confirm that the differences in myb-protein expression is not due to differences in reactivity of the monoclonal antibody but to the level of expression.

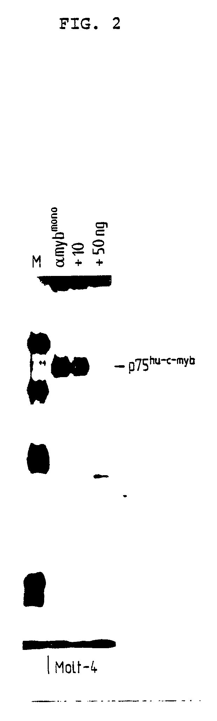

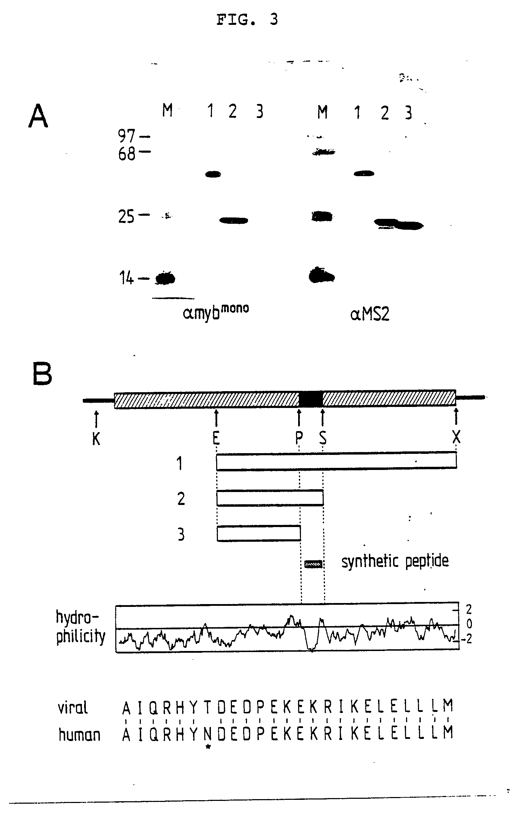

We have tried to raise an antibody against the v-myb protein by selecting one prominent hydrophilic region for synthesis of an oligopeptide assuming this molecule would be an immunogenic antigen. Hydrophilic regions are often more accessible to the immune system and therefore are assumed to play a role in the antibody response. They are identified by computer analysis of the nucleotide sequence using a computer program of Kyte and Doolittle (1982). The sequence, which was selected on that basis consists of 19 amino acids (Y T D E D P E K E K R I K E L E L L L), and turned out to be highly conserved when compared to the amino acid sequence of the human c-myb. It contained only one mismatch (position 2 N instead of T) (see Figure 3B). The oligopeptide, which did not give rise to precipitating antibodies, efficiently competed for the monoclonal antibody (Figure 2). Thus the monoclonal antibody recognized the domain made up by this oligopeptide sequence. Instead of the peptide the antigen itself or fragments thereof can

3

be used for competition analysis, in particular for monoclonal antibodies against unidentified epitopes.

The epitope recognized by the monoclonal antibody was mapped independently by using subclones of the E.coli expressed protein. Instead of the EcoRI/XbaI fragment, an EcoRI/SalI and an EcoRI/PstI fragment of v-myb were expressed as MS2-fusion proteins using again the pPLc24 as vector. The EcoRI/SalI fragment had to be subcloned into the known pUC8 vector to create suitable restriction sites for the pPLc24 vector. The molecular weight of the three fusion proteins, numbered 1, 2 and 3 in Figure 3, are 47,000, 23,000 and 21,000 dalton, respectively. Approximately 11,000 dalton molecular weight are contributed by the 99 amino acids of the fused MS-2 polymerase portion. For mapping analysis these proteins were tested in Western-blots with the hybridoma clone 4/14. Another monoclonal antibody directed against the MS-2 portion of the proteins allowed their identification. The result shown in Figure 3a indicates that clone 4/14 fails to react with fragment 3 which maps the recognition site between amino acid 208 and 232. Alignment of the v-myb gene with a computer-calculated hydrophilicity plot according to Kyte and Doolittle, 1982, supports the identity of the epitope with the most prominent hydrophilic region from which the peptide had been selected (Figure 3B).

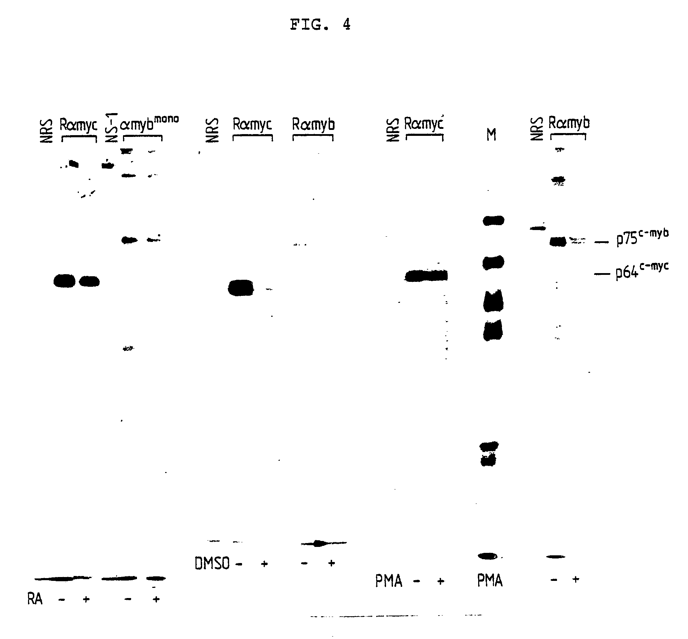

The HL-60 cell line has been described as a human promyelocytic leukaemic cell line which can be induced to differentiate by various agents. While dimethyl sulfoxide (DMSO) and retinoic acid induce HL-60 cells to differentiate into granulocytes, phorbol 12-myristate 13-acetate (PMA) treatment directs them into monocytes. Both differentiation processes have been correlated with reduction of c-myc transcripts. In the case of granulocytic differentiation a decrease of c-myb mRNA has been described as well. The monoclonal antibody cl 4/14 and a polyclonal myb-specific rabbit serum confirm these observations at the level of protein expression and extend them for the monocytic differentiation of HL-60. Cells were treated with the inducing agents, labeled metabolically with [35S]-methionine or [35S]-cysteine, and were then lysed and processed for indirect immunoprecipitation. C-myc protein expression was analyzed in parallel using a polyvalent rabbit serum. For quantitative analysis, immunoprecipitates from equivalent amounts of cell lysates, as measured by trichloroacetic acid-insoluble radioactivity, were compared. As shown in Figure 4, the effect of reduction of c-myb and c-myc protein expression is most dramatic after DMSO treatment and less significant after retinoic acid or PMA treatment. A 24h or 48h lasting retinoic acid treatment did not lead to an additional decrease of c-myb and c-myc protein expression.

The conserved and hydrophilic epitope of the human c-myb protein recognized by the monoclonal antibody of the present invention differs from another monoclonal antibody described recently which is directed against a region mapping at the very amino-terminus of the v-myb protein (Klempnauer et al., 1986).


## Immunostaining analyses of c-myb protein expression in a human tumor cell line , stimulated human lymphocytes, and fresh human lymphomas

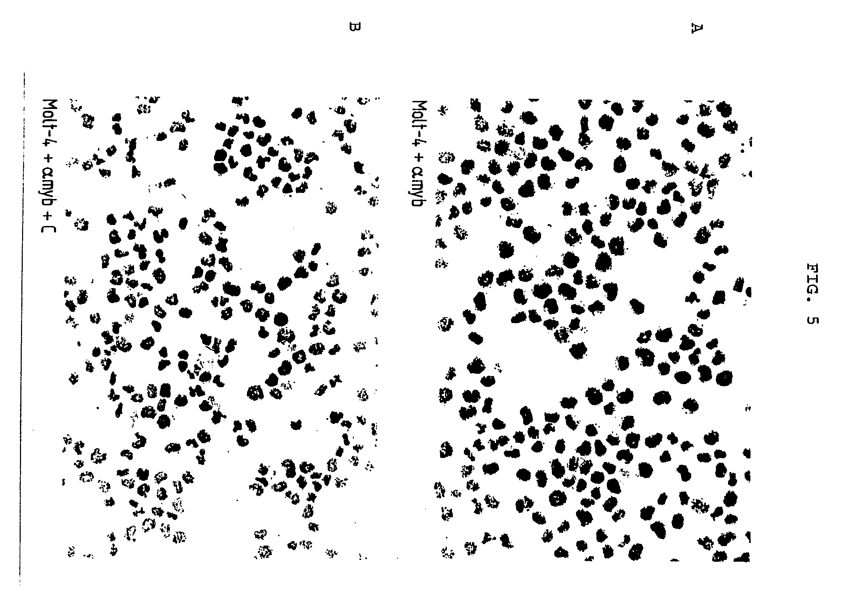

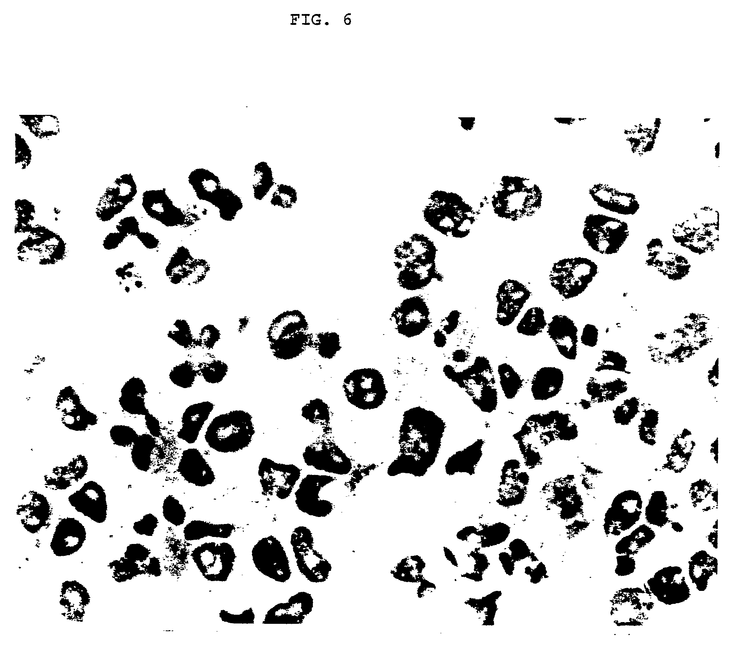

The anti-myb monoclonal antibody cl 4/14 allowed the detection of the human c-myb protein by immunocytochemical and immunohistochemical techniques, whereby no significant differences were observed between peroxidase or alkaline phosphatase as enzymes linked to the second antibody. The latter enzyme was used in the so-called APAAP sandwich technique consisting of the alkaline phosphatase-anti-alkaline phosphatase complex. Figure 5A shows an immunocytochemical analysis of Molt-4 cells using cl 4/14, which results in nuclear staining. Prior absorption of the monoclonal antibody with the bacterially expressed myb protein blocked the staining demonstrating the specificity of the reaction (Fig.5B). Immunocytochemical staining of the nucleus of avian BM-2 cells using monoclonal antibody 4/14 is shown in Fig. 6.

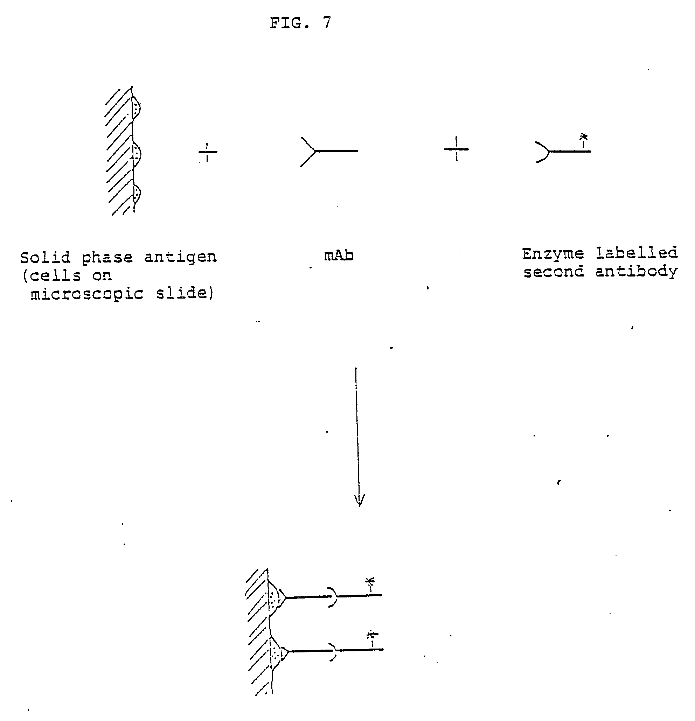

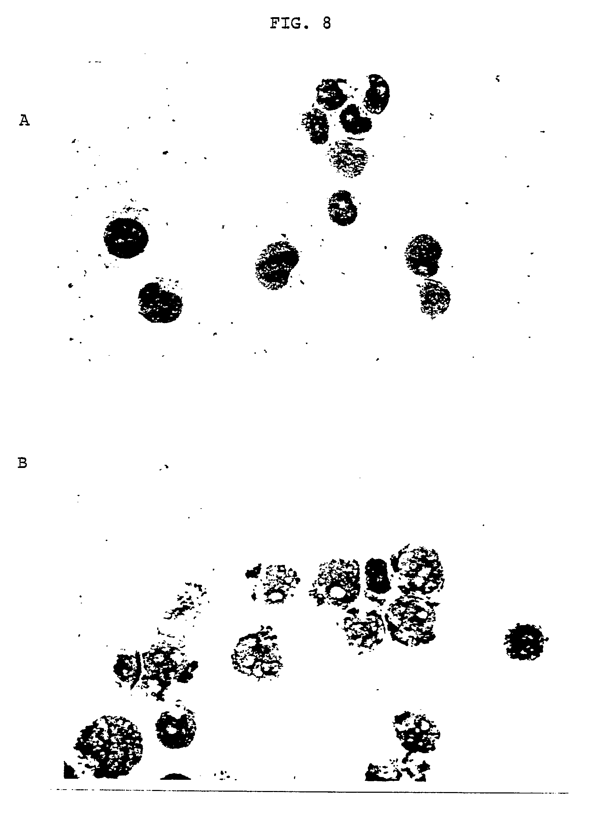

The monoclonal antibody clone 4/14 is also useful for the detection of human tumor cells from the haematopoietic and lymphatic system by fluorescence activated cell-sorting. Typical examples of tumor cells of the haematopoietic and lymphatic system are acute lymphoblastic leukaemia of B or T type, chronic lympho-cytic leukaemia, immunoblastic lymphoma, plasmoblastic lymphoma, centroblastic/centrocytic lymphoma. The immunocytochemical assay is schematically illustrated in Fig.7.

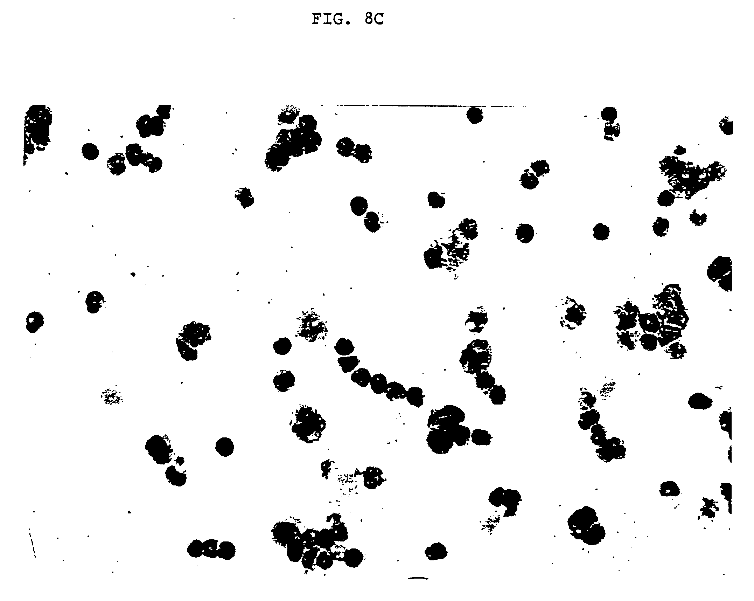

Human c-myb expression is not limited to immature proliferating cells but has also been detected at the mRNA level in fully differentiated lymphocytes if stimulated to proliferate. These analyses were confirmed at the protein level with cl 4/14 using the immunocytochemical technique. Resting T8[*] lymphocytes did not exhibit any positive staining with cl 4/14 (Figure 8A). Furthermore, unstimulated peripheral blood mononuclear cells (PBMC) as well as resting T4[*] or B-cells were negative with respect to c-myb protein expression. On day 3 after PHA stimulation, T8[*] lymphocytes exhibit strong immunostaining signals, located exclusively in the cytoplasm (Figure 8B). The same cytoplasmic staining was observed in PBMC as

4

well as T4$^+$ cells on day 3 after PHA stimulation. Staphylococcus aureus stimulated B-cells showed only a very weak staining, which was mainly restricted to the golgi region. Cytoplasmic localization of the c-myb protein in stimulated lymphocytes has been confirmed by independent techniques such as cell fractionation and use of independently derived antibodies.

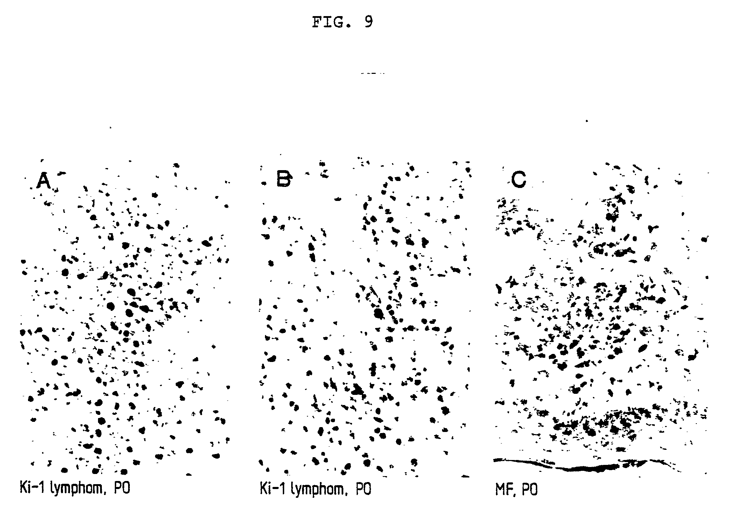

A screening of 70 human tumors of the myeloid and lymphoid system was performed by immunohistochemical staining of frozen sections using cl 4/14. In addition to the morphological characteristics of the tumors, several routine marker antibodies have been used for the tumor classification. These markers comprise the Ki-67 and Ki-1 antibody. The Ki-67 antibody lables the nuclei of all proliferating cells and thus allows the determination of the proliferative acitivity of lymphomas. The Ki-1 antibody binds to the surface of a particular anaplastic large cell non-Hodgkin's lymphoma, which originates from activated lymphoid cells of either T- or B-cell type and has been designated Ki-1 lymphoma. Figure 9A shows a Ki-1 lymphoma which expresses the c-myb protein. About 5 to 60% of the tumor cells stain positively, predominantly in the nucleus. Another Ki-1 lymphoma shown in Figure 9B contains very few myb-expressing cells and was scored as c-myb negative. All mycosis fungoides tested, were completely negative for c-myb expression (Fig.9C). With the exception of one case, the chronic lymphatic leukaemias, like mycosis fungoides, originating from small peripheral lymphoid cells and classified as "cytic" lymphoma of low proliferative activity, were negative for c-myb protein expression. Table 1 summarizes the results on c-myb protein expression in the screened tumors. The numbers indicate the ratio of positive versus total cases. Two prominent groups of lymphomas with high proliferative activity can be detected, the anaplastic large cell Ki-1 lymphomas and the lymphoblastic lymphomas of T-cell and B-cell type, one of which is shown in Fig. 8C and Fig. 9A. The immunostaining was mainly nuclear, but in some cases additionally cytoplasmic. Some cases of immunoblastic, plasmoblastic and centroblastic lymphomas were scored c-myb positive, although the immunostaining signals were rather weak. The lymphoblastic lymphomas of Burkitt type were negative for c-myb expression. Only two cases of acute myeloid leukaemia (AML) and chronic myeloid leukaemia (CML) have been analyzed so far. C-myb expression in several human AML has been shown. The AML analyzed here was considered positive for c-myb expression. For comparison and evaluation of the specificity of the antibody cells from normal tissue were tested. The connective tissue and adipocytes, ileum, appendix, and lung were all negative. Positive signals were observed in hair follicles, some activated macrophages such as epitheloid cells and lymphnode sinus macrophages, some endothelial cells, epidermis (only in some cases) and in endometrical secretory cells, where staining was cytoplasmic. Very few hepatocytes and secretory pancreatic cells also exhibited a faint cytoplasmic staining.

The monoclonal antibodies of the present invention can be used therapeutically.

Treatment of disease states (e.g. malignancies) manifesting themselves as an overexpression of c-myb protein may be accomplished by administering a therapeutically effective amount of 4/14 or other monoclonal antibodies to an individual in need of such treatment. The monoclonal antibody of the present invention may be used by parenterally administration. As an alternative isolated bone marrow may be depleted from the tumor cells using transient in vitro culture in the presence of the monoclonal antibody. The invention is further illustrated by the following examples.

The BM-2 line of chicken myeloblasts transformed by AMV and the KG-1 and HL-60 lines have been described. Molt-4 and Colo 205 are human T-lymphoblastic cell lines obtainable from ATCC, Rockville, Maryland, No. 1582 and CCL 222.

BM-2 was grown in DMEM medium supplemented with 1 mM sodiumpyruvate, essential amino acids, vitamine, 8% fetal calf serum (FCS), and 2% heat-inactivated chicken serum. The human cell lines COLO 205, HL-60, KG-1, and Molt-4 were maintained in RPMI medium containing 10% FCS. All media were supplemented with 100 units per ml of penicillin, 200 μg per ml streptomycin, and 2 mM glutamine. The cells were cultured at 37° C with 5% $CO_2$ atmosphere.

Metabolic labeling has been performed in methionine-free medium supplemented with 5% dialyzed calf serum and 500 μCi/ml of [$^{35}$S]-methionine (> 800 Ci/mmole, Amersham, England) for 90 min or in cysteine-free medium containing 5% dialyzed calf serum and 500 μCi per ml of [$^{35}$S]-cysteine (>800 Ci/mmole , Amersham, England) for 60 min.

Fresh unfixed biopsies of lymphoma tissues were obtained from the Klinikum Steglitz, Free University Berlin. The tissues were frozen in airtight plastic capsules by immersion in liquid nitrogen and stored at -70° C for up to three years before use. All lymphoma cases were classified according to conventional morphological, enzyme cytochemical, and immunohistological criteria, using polyclonal and monoclonal antibodies. In all cases the clinical findings were consistent with the morphological diagnosis.

Peripheral blood mononuclear cells (PBMNC) were prepared by Ficoll-Hypaque (Pharmacia, Uppsala, Sweden) density centrifugation of heparinized blood from healthy donors. Purified T-lymphocytes were isolated from PBMNC's by erythrocyte rosette formation using 2-aminoethyl-isothiouronium-bromide hydrobromide (AET)-treated sheep red blood cells and Ficoll-Hypaque density centrifugation. Separation of T-lymphocytes into T4[+] and T8[+] subsets was performed as described. B-cell enriched cell populations were obtained after two steps of erythrocyte rosetting and subsequent removal of monocytes by plastic or glass adherence.

Example 1

Preparation of Monoclonal Antibodies

E.coli expressed MS2-v-myb fusion protein (Moelling et al. 1985, and 1987) was used for immunization of mice. Six Balb/c mice were immunized repeatedly with gel-eluted bacterial protein. All animals exhibited high-titered antibodies. One animal was sacrificed three days after a final intraperitoneal immunization. The NS-1 myeloma cell line was used for the fusion essentially as described (Greiser-Wilke et al., 1981). 600 colonies gave rise to 18 positive clones, twelve of which were maintained stably in culture after several rounds of end-point dilution subcloning. Positive cultures were screened by ELISA and Western-blot using the bacterially expressed antigen and by indirect immunoprecipitation using metabolically labeled BM-2 and Molt-4 cells. Clone 4/14 was the only clone crossreactive with the human c-myb protein in the indirect immunoprecipitation.

Example 2

Indirect Immunoprecipitation

Exponentially growing cells of the human T-lymphoblastic cell lines Molt-4, KG-1 and Colo 205 and the AMV-transformed avian bone marrow cell line BM-2 were labeled metabolically with 500 $\mu$Ci/ml of [$^{35}$S]-methionine in methionine-free medium containing 5% dialyzed calf serum for 90 min. Cells were washed twice with cold phosphate-buffered saline (PBS: 8 mM $Na_2HPO_4$, 1.5 mM $KH_2PO_4$, pH 7.4. 3 mM KCl, 140 mM NaCl) and lysed at 4°C in lysis buffer (10 mM Tris-HCl, pH 7.5, 50 mM NaCl, 0.5% NP-40, 0.5% sodium desoxycholate (DOC), 0.5% sodium dodecylsulfate (SDS), 0.5% Trasylol (Bayer Co., Ludwigshafen, West Germany), 1 mM phenyl-methyl-sulfonyl-fluoride (PMSF)). The lysate was passed seven times through a 25G needle to shear the DNA and then centrifuged at 10.000xg for 30 min at 4°C. 0.2 ml lysate, equivalent to 2x10^6 cells, was treated with antiserum (5 $\mu$l) or hybridoma supernatants (200 $\mu$l) or ascites fluid (10 $\mu$l) and Staphylococcus aureus (Pansorbin, Calbiochem-Behring). Purified MS2-v-myb protein (5 $\mu$g) which had served as antigen for immunization was used as competing protein during the precipitation. The immunocomplexes obtained were washed extensively and analyzed by gel electrophoresis (10% gels). The gel was soaked in a solution consisting of 1M sodium-salicylate for 30 min at room temperature, dried and exposed for autoradiography at -70°C. Competition experiments with synthetic peptides were performed using 10 and 50 ng. The synthetic peptide was purchased from Biochrom KG, Berlin-West.

Figure 1 shows an indirect immunoprecipitation of the human c-myb protein (p75c-myb) and the viral myb protein (p48v-myb). The antibodies used are: normal rabbit serum (1); rabbit anti-v-myb serum (2); control supernatant from myeloma cells (3); anti-myb monoclonal antibody (supernatant) (4); anti-myb monoclonal antibody (supernatant) blocked with bacterially expressed myb protein (5). M indicates molecular weight marker, from top to bottom: 92 kDa, 68 kDa, 55 kDa, 45 kDa, 32 kDa. Exposure time: 1 week.

Figure 2 shows the competition of the immunoprecipitation reaction by a synthetic v-myb specific peptide. Indirect immunoprecipitation of Molt-4 lysates was performed using 10 $\mu$l of ascites fluid from hybridoma clone 4/14 ($\alpha$myb$^{mono}$). Indicated amounts of synthetic v-myb specific peptide of 19 amino acids (213 to 231) was added as competing antigen blocking the precipitation reaction. Exposure time: 7 days.

M indicates molecular weight markers, from top: 92K, 68K, 54K (faint), 45K, 32K.

Example 3

a) Construction of truncated myb fusion proteins

To identify the myb epitope recognized by monoclonal antibody 4/14 different fragments of the v-myb coding region, numbered, 1, 2 and 3 (see Fig. 3B) were expressed as MS2-fusion proteins in E.coli. Fragment 1 corresponds to the EcoRI/XbaI (E/X) region, 2 to EcoRI/SalI (E/S), and 3 to EcoRI/PstI (E/P). Besides the EcoRI/XbaI fragment the EcoRI/SalI fragment and the EcoRI/PstI fragment were cloned into a derivative of the known thermoinducible MS2 expression vector pPLc24 which contained a polylinker. The EcoRI/SalI fragment had to be subcloned into the known pUC8 vector to create suitable restriction sites for pPLc24 plasmid. The three MS2-fusion proteins which contain 99 MS2 polymerase-specific amino acids (equivalent to about 11,000 dalton), have molecular weights of 47,000, 23,000 and 21,000 dalton, respectively.

b) Epitope mapping of monoclonal antibody 4/14 by Western-blot analysis

Bacterially expressed proteins were analyzed by Western-blot analysis (see Fig. 3A). The equivalent of about 100 $\mu$g of bacterial lysate was electrophoresed (12.5% gel), and transferred to nitrocellulose (60V, 2.5 hrs) in transfer buffer (0.069 M glycine, 12.5 mM. Trismabase, 0.05% SDS, 20% methanol). Blots were blocked by incubation with 5% non-fat dried milk in PBS and 0.3 % Tween 20 (PBS/T/M buffer) for 30 min at room temperature (RT). Subsequently the sheet was incubated with ascites fluid from clone 4/14 or anti-MS2, diluted 1:500 in PBS/T/M buffer for 1 hr at RT. Non-specific binding was reduced by further treatment with PBS containing 0.1% SDS and two washes with PBS/T buffer. Goat anti-mouse immunoglobulin alkaline phosphatase conjugate (1:1000 dilution in PBS/T/M buffer) was added for 1 hr at RT, followed by repeated washing with PBS/T buffer. Alkaline phosphatase uses 5-bromo-4-chloroindolyl phosphate as substrate which results in a multiple step reaction in reduction of nitroblue-tetrazolium to diformazan which gives rise to a red color. M indicates molecular weight markers indicated in kilodalton. In Fig. 3B the hatched box schematically indicates the complete v-myb gene with flanking viral regions and a KpnI (K) restriction site. 1, 2 and 3, the three bacterially expressed v-myb proteins also shown in (A) are schematically drawn to scale (open boxes) and a hydrophilicity curve using the computer program of Kyte and Doolittle (1982) was lined up with the v-myb sequence. Hydrophilicity is indicated by negative number. The black box (P/S) corresponds to the epitope exclusively recognized by clone 4/14 which lines up with a prominent hydrophilic peak. Its amino-acid sequence is listed at the bottom (A through M) and compared to the human c-myb amino acid sequence
(* indicates a mismatch). This sequence homology between v-myb and human c-myb is indicative for a conserved epitope. During evolution only those sequences are conserved which serve important molecular functions. The synthetic peptide is indicated as dotted box, its amino-acid sequence ranges from Y to the last but one amino acid (L).

Example 4

Immunocytochemical analysis of c-myb protein expression in human leukaemias and lymphomas using hybridoma clone 4/14.

Leukaemic cells, obtained from peripheral blood or bone marrow aspirates were enriched by centrifugation on standard Ficoll-Paque cushions (Pharmacia, Uppsala, Sweden). The mononuclear cells were washed twice with phosphate-buffered saline (PBS) and subsequently $1 \times 10^5$ to $2 \times 10^5$ were cytocentrifuged (10 min 800xg) onto microscopic slides. In the case of established cell lines of human origin the cells are directly cytocentrifuged onto the slides. The cells were air-dried and fixed in acetone for 15 min. The fixed slides were incubated for 30 min at room temperature with either undiluted hybridoma supernatant or ascites fluid

7

diluted between 1:100 and 1:500. After incubation with monoclonal antibody the slides were washed in PBS. For the indirect immunoperoxidase method they were then treated for 30 min with peroxidase-conjugated rabbit anti-mouse Ig (Dako, Copenhagen, Denmark) diluted 1:10 in PBS. After a short wash (1 min) with PBS the slides were stained with diaminobenzidine (0.6 mg/ml) and hydrogen peroxide (0.01%) for 10 min at room temperature and finally washed with PBS. For the APAAP sandwich technique the slides were washed with TBS and then incubated with rabbit anti-mouse immunoglobulin (Ig) serum (Dakopatts Co., Copenhagen, Denmark) for 30 min and subsequently treated with alkaline phosphatase-anti-alkaline phosphatase (APAAP) complexes for 30 min as described by Cordell et al. (1984). Incubation with rabbit antimouse Ig serum and APAAP was repeated twice, and thereafter, the alkaline phosphatase was visualized with the modified New Fuchsin method. Competition experiments were performed using an excess of bacterially expressed myb protein to block the myb monoclonal antibodies prior to incubation.

For staining of fresh tissues, frozen tissue sections were transferred to microscopic slides, fixed in acetone and subsequently in chloroform each for 15 min at room temperature.

All following steps were performed as described above for the immunostaining of leukaemic cells from peripheral blood, bone marrow aspirates or cell lines. The slides are examined under a microscope at a magnification of 150 to 800. Figure 5 shows the immunocytochemical analysis of Molt-4 cells using ascites fluid of the anti-myb monoclonal antibody (αmyb) and the peroxidase staining technique. To compete for the immunostaining bacterially expressed myb protein was used for blocking the antibody as indicated by (+C).

Figure 9 shows the immunohistochemical staining of frozen sections from two human large cell Ki-1 lymphomas and a mycosis fungoides (MF) using ascites fluid of the anti-myb monoclonal antibody and the peroxidase technique (PO).

Immunostaining of a series of human leukaemias and lymphomas with hybridoma clone 4/14 indicate high c-myb protein expression in T and B precursor ALL (acute lymphoblastic leukaemia) as well as in several non-Hodgkin's lymphomas (see also Table I).

Example 5

Fluorescence activated cell sorting of c-myb protein expressing human leukaemias and lymphomas using hybridoma clone 4/14.

Leukaemic cells obtained from peripheral blood or bone marrow aspirates or single-cell suspensions of lymphatic tissues are centrifuged, washed twice in PBS, treated with 95% acetone for 10 min at room temperature and resuspended in PBS ($10^7$ cells/ml). 100 µl are processed for staining using 100 µl of undiluted hybridoma supernatant of clone 4/14. After 45 min at room temperature samples are centrifuged and the pellets incubated in 10 µl of fluorescein isothiocyanate-conjugated rabbit immuno-globulin (Sigma Co, München). Fluorescence assays are carried out with standard flow cytometer. An argon laser tuned to the 488 nm line at a light power of 100 mW is used to excite green fluorescence from the fluorescein-tagged immunoglobulin. The fluorescence signal from each cell is quantitated by photodetector. The number of myb-expressing cells is compared with the total cell number input by cell-counting (Coulter Counter). Fluorescence labeling of the cells by using other monoclonal antibodies against cell-surface markers or against lymphoma-specific antigens (e.g. Ki-1) applying the same procedure allow a differential diagnosis of a patient's disease.

Example 6

Induction of differentiation of HL-60 cells.

Exponentially growing HL-60 cells were seeded at a density of 2x10⁵ cells per ml and treated for 6 h with either 1.2% dimethyl sulfoxide (DMSO) or $10^{-6}$ M retinoic acid (Sigma, diluted from a $10^{-3}$ M ethanol stock). Phorbol 12-myristate 13-acetate (PMA) induction was carried out with 40 nM PMA (Sigma, diluted from a 1 mg per ml ethanol stock) for 24 h. Subsequently the cells were washed with phosphate-buffered saline (PBS) and metabolically labeled in the presence of the inducing agent. The cells were then processed for indirect immunoprecipitation as described in Example 2.

Figure 4 shows the indirect immunoprecipitation of the human c-myb protein (p75-myb) and the human c-myb protein (p64c-myc) from HL-60 cells with (+) or without (-) treatment with retinoid acid (RA), dimethyl sulfoxide (DMSO), or phorbolester (PMA). Immunoprecipitation was performed with normal rabbit serum (NRS), rabbit anti-v-myc and rabbit anti-v-myb serum (Rαmyc, Rαmyb), control supernatant from myeloma cells (NS-1), and ascites fluid from anti-myb monoclonal antibody (αmyb^mono). M: molecular weight marker as described in Figure 1. Exposure time: 3-10 days.

Example 7

Mitogen stimulation

Stimulation by phytohaemagglutinin (PHA) was performed using PBMNC's, purified T-lymphocytes, T4⁺ or T8⁺ cells, which were seeded at a concentration of 10⁶ per ml in RPMI medium supplemented with 10% FCS, antibiotics, and 1 µg per ml PHA (Gibco). Cell suspensions were processed for immunocytochemical staining by cytocentrifugation (Shandon Co.) onto slides on days 0, 1, 2, and 3 after PHA treatment. Stimulation by Staphylococcus aureus was performed using the B-cell enriched population, which was seeded at a concentration of 2x10⁵ per ml in RPMI medium containing $2x10^{-5}$ (v/v) formalin-fixed Staphylococcus aureus Cowan I bacteria. The cells were harvested, submitted to cytocentrifugation, and processed for immunocytochemical staining on days 0 , 1, 2, and 3. Figure 8 shows the immunocytochemical staining of human T8⁺ cells before (A) and on day 3 after PHA stimulation (B) and a lymphoblastic lymphoma of pre B-cell type (C) using ascites fluid of the anti-myb monoclonal antibody and the APAAP procedure.

The hybridoma cell line clone 4/14 was deposited according to the Budapest Agreement with NCACC, Porton Down, England on 22.6.1987 and received the deposition No. 87062301.

References

Cordell, J.L., Falini, B., Erber, W.N., Ghosh, A.K., Abdulaziz, Z., Macdonald, S., Pulford, K.A.F., Stein, H., and Mason, D.Y. (1984) J. Histochem. Cyto-chem. 32, 219-229.
Galfré, G. and Milstein, C. (1981) Methods in Enzymology, Vol.73, 3 et seqq.
Greiser-Wilke, I., Owada, M.K. and Moelling, K. (1981) J. Virol., 39, 325-329.
Klempnauer, K.-H., Bonifer, C. and Sippel, A.E. (1986) EMBO J., 5, 1903-1911.
Kyte, J. and Doolittle, R.F. (1982) J. Mol. Biol., 157, 105-132.
Moelling, K., Heimann, B., Beimling, P., Bepler, G., Köppler, H., Havemann, K. and Balzer, T. (1987) Contr. Oncol., Vol.24, 76-86.
Moelling, K., Pfaff, E., Beug, H., Beimling, P., Bunte, T., Schaller, H.E. and Graf, T. (1985) Cell, 40, 983-990.

Table I

Reactivity of lymphoid and myeloid malignancies with monoclonal antibody against c-myb

NHL with high proliferative activity                    % pos. cells

| | | |
|---|---|---|
| Lymphoblastic of Burkitt type | 0/3 | |
| Lymphoblastic of pre B-cell type | 9/10 | 10-80 |
| Lymphoblastic of T-cell type | 3/4 | 10-70 |
| Immunoblastic of B-cell type | 2/5 | 70 |
| Plasmoblastic lymphoma | 2/2 | 30; 70 |
| Centroblastic lymphoma | 1/4 | 1 |
| Large cell Ki-1 lymphoma of T-type | 5/8 | 5-60 |
| Large cell Ki-1 lymphoma of O-type | 0/1 | |

NHL with intermediate proliferative activity

| | | |
|---|---|---|
| Pleomorphic T-cell lymphoma | 0/2 | |
| T-cell lymphoma of angio immunoblastic lymphadenophathy | 0/2 | |

NHL with low proliferative activity

| | | |
|---|---|---|
| Centroblastic/centrocytic lymphoma | 2/4 | < 1; 5 |
| lymphocytic plasma cell immunocytoma | 2/5 | < 1 |
| B-cell chronic lymphocytic | | |

| leukaemia mycosis fungoides | 1/5 | 1 |
|---|---|---|
| mycosis fungoides | 0/9 | |

**Myeloid leukaemias**

| Acute myeloid leukaemia | 1/1 | 30 |
|---|---|---|
| chronic myeloid leukaemia | 0/1 | |

**Other lymphomas**

| Hodgkin's lymphoma | 0/4 |
|---|---|

---

Immunohistochemical staining was performed with peroxidase or APAAP
(NHL = non-Hodgkin's lymphoma).

## Claims

1. Hybridoma cell line secreting a monoclonal antibody reactive with an epitope which is hydrophilic and/or conserved in the c-myb proteins of vertebrates.

2. Hybridoma cell line according to claim 1 secreting a monoclonal antibody reactive with both the viral and the human c-myb proteins and recognizing on the human c-myb protein an epitope comprising the following amino acid sequence:

A I Q R H Y N D E D P E K E K R I K E L E L L L M

3. Monoclonal antibody reactive with an epitope which is hydrophilic and/or conserved in the c-myb proteins of vertebrates.

4. Monoclonal antibody reactive with both the viral and the human c-myb protein and recognizing on the human c-myb protein an epitope comprising the following amino acid sequence:

A I Q R H Y N D E D P E K E K R I K E L E L L L M

5. A method for immunocytochemical detection of c-myb protein expression in human tissue, cells or cell lines comprising the use of the monoclonal antibodies according to claim 3 or 4 in an immunocytochemical reaction with a sample suspected of containing said tissue, cells or cell lines.

6. A method according to claim 5 wherein the cells are cells of the hematopoietic and lymphatic system.

7. A method for detection of c-myb protein expression according to claim 5 or 6 by using fluorescence activated cell sorting (FACS).

8. A method for detection of c-myb protein expression according to claim 5 or 6 comprising the use of ELISA techniques, Western-Blot techniques or indirect immuno-precipitation.

9. A kit for the detection of c-myb in human tumor cells comprising a monoclonal antibody according to claim 3 or 4, a control antibody, wash buffers, a second polyclonal or monoclonal antibody which is radioactively tagged or tagged with an indicator such as peroxidase or alkaline phosphatase and substrate.

10. A pharmaceutical composition comprising an effective amount of a monoclonal antibody according to claim 3 or 4 in combination with a pharmaceutically acceptable carrier for eliminating cells overproducing c-myb protein.

FIG. 1

System

FIG. 2

M  αmyb$^{mono}$  +10  +50 ng

— p75$^{hu-c-myb}$

| Molt-4

EP 0 298 450 A2

FIG. 3

A

M   1 2 3    M   1 2 3

97—
68—

25—

14—

αmyb^mono          αMS2

B

K          E        P  S              X

1

2

3

synthetic peptide

hydro-
philicity

viral   A I Q R H Y T D E D P E K E K R I K E L E L L L M
human   A I Q R H Y N D E D P E K E K R I K E L E L L L M

## FIG. 4

FIG. 5

A

Molt-4 + αmyb

B

Molt-4 + αmyb + C

EP 0 298 450 A2

FIG. 6

FIG. 7

Solid phase antigen          mAb          Enzyme labelled
(cells on                                 second antibody
 microscopic slide)

FIG. 8

A

B

FIG. 8C

FIG. 9

Ki-1 lymphom, PO          Ki-1 lymphom, PO          MF, PO